Europäisches Patentamt

European Patent Office

Office européen des brevets

(11) Veröffentlichungsnummer: **0 330 138**
**A1**

## (12) EUROPÄISCHE PATENTANMELDUNG

(21) Anmeldenummer: 89102982.9

(22) Anmeldetag: 21.02.89

(51) Int. Cl.⁴: **B65F 1/00 , B65D 5/24 , B65D 5/44 , B65D 5/64**

(30) Priorität: 25.02.88 DE 3805914

(43) Veröffentlichungstag der Anmeldung:
30.08.89 Patentblatt 89/35

(84) Benannte Vertragsstaaten:
AT BE CH DE ES FR GB GR IT LI LU NL SE

(71) Anmelder: KLINGELE PAPIERWERKE GMBH &
CO

D-7064 Remshalden(DE)

(72) Erfinder: Waldenmeier, Werner
Amselweg 19
D-7064 Remshalden-Geradstetten(DE)
Erfinder: Zimmermann, Franz
Lilienweg 5
D-7060 Schorndorf(DE)
Erfinder: Lehmair, Michael
Biedersbergweg 68
D-6680 Neunkirchen(DE)
Erfinder: Rospert, Artur
Provinzial-Strasse 83
D-6610 Lebach(DE)

(74) Vertreter: Glöser, Otto
Sudetenstrasse 6
D-8076 Baar-Ebenhausen 1(DE)

(54) **Abfallbehälter.**

(57) Die Erfindung bezieht sich auf einen Abfallbehälter, insbesondere für ansteckungsgefährliche Abfälle oder für sonstige gefährliche Stoffe, der eine beliebige Gestalt aufweist und mit einem Deckel verschließbar ist. Bei einem solchen Behälter ist angestrebt, daß er den einschlägigen Bestimmungen der Gesundheitsbehörden und des Umweltschutzes genügt. Dies wird dadurch erreicht, daß ein einziger aus einem Werkstoff auf Holzbasis, z.B. aus Wellpappe hergestellter Zuschnitt (1) um Faltlinien (3 - 6, 11 - 18 und 23 - 26) zu dem Behälter (31) aufstellbar ist, wobei der Zuschnitt (1) zumindest an derjenigen Fläche eine flüssigkeitsdichte Schicht (vgl. 35), Beschichtung, Imprägnierung o. dgl. aufweist, welche die Boden- und Innenflächen des Behälters (31) ergibt, und die von einem z.B. quadratischen Bodenfeld (2) nach oben strebenden Eckbereiche (36 - 39) des Behälters (31) mit einer flüssigkeitsdichten Masse, insbesondere mit einer klebenden Dichtungsmasse ausgekleidet sind und der obere Rand des Behälters (1) für die Aufnahme eines abdichtenden Deckels (40, 50) hergerichtet ist.

Fig.1

## Abfallbehälter

Die Erfindung bezieht sich auf einen Abfallbehälter, insbesondere für ansteckungsgefährliche Abfälle oder für sonstige gefährliche Stoffe, der eine beliebige Gestalt aufweist und mit einem Deckel verschließbar ist.

Abfallbehälter sind in mannigfachen Ausführungen bekannt und sie werden in der Regel aus Kunststoff oder aus mit Kunststoff versetzten und/oder imprägnierten Materialien hergestellt. Derartige Behälter erfüllen ihren Zweck, jedoch treten dann Probleme auf, wenn sie schadhaft werden und ausgesondert werden müssen. Da die Ablagerung auf Deponien viel Raum benötigt, der meistens nicht zur Verfügung steht, ist man dazu übergegangen, die Behälter und auch sonstige aus Kunststoffen bestehende Materialien zu verbrennen, wodurch zwar keine nicht verrottenden Halden entstehen, dafür aber Schadstoffe jeglicher Art freige setzt werden, welche die Umwelt als solche, insbesondere die Luft, erheblich belasten, was heute nicht mehr hingenommen werden kann. Letzteres gilt vor allem für Abfallbehälter, die Abfälle der eingangs erwähnten Art, z.B. aus Krankenhäusern, beinhalten und dicht verschlossen einer Verbrennungsanlage und in vielen Fällen sogar einer Sondermüllbeseitigungsanlage zugeführt werden müssen. Zwar sind bereits Behälter aus Wellpappe für die genannten Problemabfälle vorgeschlagen worden, jedoch werden diese Erzeugnisse nicht den Bestimmungen der GGVS und der GGVE gerecht. Dies aber nicht nur wegen unzureichender Dichtigkeit, sondern auch wegen mangelnder Festigkeit, die nicht ausgereicht hat, um die gefüllten Behälter ohne auf den Inhalt zurückgehende Gefahr für Tier und Mensch über längere Strecken zu einer Beseitigungsanlage zu transportieren.

Demgegenüber liegt der Erfindung die Aufgabe zugrunde, einen insbesondere für ansteckungsgefährliche Abfälle geeigneten Abfallbehälter zu schaffen, der einmal ausreichend fest und flüssigkeitsdicht ist und zum anderen möglichst wenige derjenigen Materialien aufweist, die beim Verbrennen Schadstoffe freisetzen. Anders ausgedrückt bedeutet dies, daß ein Abfallbehälter anzustreben ist, der den einschlägigen Bestimmungen der Gesundheitsbehörden und des Umweltschutzes genügt.

Diese Aufgabe wird mit den aus dem kennzeichnenden Teil des Anspruches 1 ersichtlichen Maßnahmen gelöst. Da der erfindungsgemäße Behälter aus einem schadlos zu beseitigenden Werkstoff besteht, ist seine Beseitigung problemlos, zumindest aber günstiger als früher, und dies auch dann, wenn der Inhalt als solcher Beseitigungsschwierigkeiten mit sich bringen könnte. Auch die Herstellung des Behälters ist gemessen an den aufwendigen Formen für Kunststoffbehälter außerordentlich günstig, zumal sowohl das Herstellen eines Zuschnittes als auch sein Aufstellen einfach ist und in kürzester Zeit vollzogen werden kann. Die durch eine besondere Behandlung erzielte Flüssigkeitsdichtigkeit ist als wesentlicher Vorteil unverkennbar. Auch den Faltlinien kommt eine große Bedeutung zu, da durch sie keinerlei Beschädigungen der Beschichtung o. dgl. auftreten, also die Dichtigkeit nicht gefährdet wird. Die zunächst als Schwachstellen anzusehenden Eckbereiche verlieren dadurch ihre Gefährlichkeit, daß sie mit einer klebenden Dichtungsmasse ausgekleidet sind, die noch dazu zu einer erhöhten Stabilität des ganzen Behälters führt. Der abdichtende Deckel - auf den noch näher eingegangen wird -schließlich schafft zusammen mit dem eigentlichen Behälter eine Einheit, die befüllt - auch mit ansteckungsgefährlichen und gfs. gewichtigen Abfällen - gefahrlos für die Umwelt einem Entsorgungsbetrieb zugeführt werden kann, wobei noch als transport- und handhabungsgünstig herauszustellen ist, daß ein z.B. aus mehrlagiger Wellpappe hergestellter Behälter ein geringes Leergewicht hat. Nicht unerwähnt soll ferner bleiben, daß Wellpappe in zahlreichen Aufbauformen zur Verfügung steht, die unterschiedliche Festigkeitseigenschaften aufweisen, umweltfreundlich sind und mit einem geringen Aufwand flüssigkeitsdicht gemacht werden können, wie dies der kennzeichnende Teil des Anspruches 1 vorschreibt. Ähnliches gilt auch für Pappe oder andere auf Holzbasis beruhende Werkstoffe.

Ein einfach aufzustellender Abfallbehälter ergibt sich dann, wenn man sich die Lehre des Anspruches 2 zunutze macht. Ein solcher Zuschnitt läßt deutlich erkennen, daß man ganz bewußt Faltlinien zur Anwendung bringt, um die Dichtigkeit an den Knickstellen nicht zu gefährden. Die Zwischenfelder, deren Teile beim aufgestellten Zuschnitt aufeinander zu liegen kommen, ergeben eine wirkungsvolle Verstärkung zweier Seitenwandfelder, ganz abgesehen davon, daß diese Zwischenfelder den Querschnitt des aufgestellten Behälters formstabil halten. Letzteres um so mehr, wenn man die Maßnahme des Anspruches 3 zur Anwendung bringt.

Da um Faltlinien verformte Wellpappezuschnitte zum Auffedern neigen, erscheint es zweckmäßig, sich der Maßnahme nach Anspruch 4 zu bedienen.

Eine weitere vorteilhafte Besonderheit der Erfindung ist dann gegeben, wenn man die Lehre nach Anspruch 5 zur Anwendung bringt. Dadurch sind nicht nur die oberen Schnittkanten des Behälters geschützt und armiert, was besonders wirksam wird, wenn man nach Anspruch 6 vorgeht. Wenn auch hier neben Metall bzw. Blech Kunststoff zur Anwendung kommt, so geschieht dies in verhältnismäßig geringen Mengen, die hingenommen werden können, wenn man sie den benötigten Materialmengen für ganz aus Kunststoff hergestellten Behältern gegenüberstellt, die früher oder später zu entsorgen sind. Metallische Rahmen bereiten beim Verbrennen keine größeren Schwierigkeiten als Blechdosen, Blechgebinde o. dgl., die in weit größeren Mengen anfallen und von den Entsorgungsanlagen verkraftet werden müssen.

Ein im oberen Bereich in der angegebenen Weise hergerichteter eigentlicher Behälter fördert auch das dichte Verschließen mit dem bereits erwähnten Deckel, der naturgemäß ähnliche Eigenschaften wie der eigentliche Behälter besitzen muß. Dies ist dann zu erreichen, wenn man die Maßnahmen nach Anspruch 7 verwirklicht. Hier sei jedoch bemerkt, daß der Deckel nach Anspruch 7 nicht unbedingt mit einem Rahmen am eigentlichen Behälter zusammenwirken muß, sondern auch auf einer anderen Abdichtung, z.B. auf einem die oberen Ränder übergreifenden Klebeband sein Widerlager finden kann. Auf jeden Fall weist auch der Deckel nach Anspruch 7 lediglich so viel Kunststoff auf, wie unbedingt erforderlich ist. Die großen Flächen bestehen nach wie vor aus einem unschädlich zu beseitigenden Werkstoff.

Für das Festlegen des Deckels auf dem Rahmen bzw. der Abdichtung des Behälters gibt es verschiedene Möglichkeiten, wie solche z.B. in den Ansprüchen 8, 9 und 10 angegeben sind. Es versteht sich, daß auch andere Verbindungsarten möglich erscheinen, ohne den Rahmen der Erfindung zu verlassen.

Ein wirtschaftlich herstellbarer Deckel ist im Anspruch 11 angegeben, während der Anspruch 12 einen Weg weist, wie man den Wellpappeanteil mit dem Kunststoff- oder Metall- bzw. Blechteil flüssigkeitsdicht verbinden kann. Es versteht sich, daß beim Verbinden der Bestandteile nicht unbedingt von den Lehren des Anspruches 12 auszugehen ist, sondern auch andere Verbindungsarten gewählt werden können, wenn gewährleistet ist, daß die absolute Dichtigkeit nicht leidet.

In der Zeichnung ist die Erfindung beispielsweise veranschaulicht; es zeigen:

Fig. 1 einen flachliegenden Zuschnitt für den erfindungsgemäßen Abfallbehälter;

Fig. 2 den zu einem Behälter aufgestellten Zuschnitt nach Fig. 1, mit einem dazugehörigen oberen Rahmen in schaubildlicher Darstellung;

Fig. 3 eine Erläuterungsansicht von oben auf den oberen Rand des aufgestellten Zuschnittes;

Fig. 4 einen flachliegenden Zuschnitt für einen Behälterdeckel;

Fig. 5 eine schaubildliche Darstellung des aufgestellten Deckelzuschnittes;

Fig. 6 eine schaubildliche Darstellung eines Deckelrahmens mit teilweise eingesetztem Zuschnitt und darunterliegendem aufgestellten Zuschnitt und

Fig. 7 einen gegenüber den anderen Figuren vergrößerten Erläuterungsquerschnitt zur Verdeutlichung der Rahmen und des Zusammenwirkens zwischen Deckel und eigentlichem Behälter.

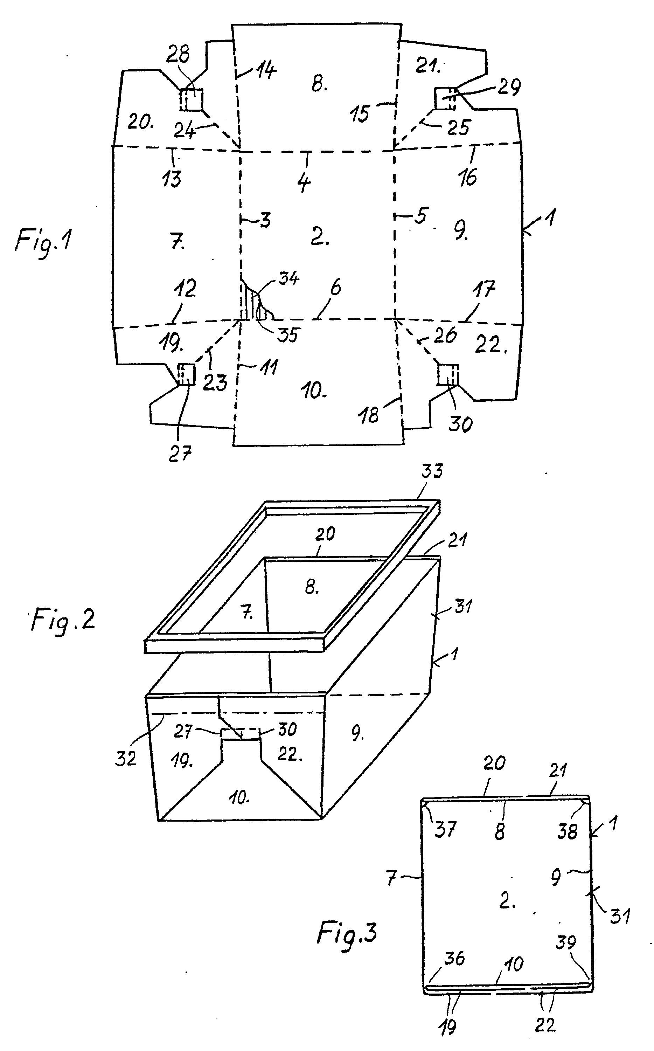

Wie sich aus Fig. 1 ergibt, weist ein Zuschnitt 1 ein Bodenfeld 2 auf, das über Faltlinien 3, 4, 5 und 6 in Seitenwandfelder 7, 8, 9 und 10 übergeht. Diese Seitenwandfelder 7 bis 10 wiederum gehen über Faltlinien 11, 12, 13, 14, 15, 16, 17 und 18 in Zwischenfelder 19, 20, 21 und 22 über. Die Zwischenfelder 19 bis 22 sind an ihren Rändern ausgespart und sie weisen diagonale Faltlinien 23, 24, 25 und 26 auf. Des weiteren sind sie mit angestanzten Sicherungslappen 27, 28, 29 und 30 ausgestattet.

Der so gestaltete Zuschnitt 1 läßt sich aufgrund der Faltlinien 3 bis 6 und 11 bis 18 sowie 23 bis 26 in die Gestalt der Fig. 2 bringen, in welche die für das Verständnis erforderlichen Bezugszahlen eingetragen sind. Das gleiche gilt für die Fig. 3, bei der die Konizität des aus dem Zuschnitt 1 aufgestellten Abfallbehälters 31 der Deutlichkeit wegen vernachlässigt ist. Ähnliches gilt für den oberen Teil der Darstellung, wo die Doppelung der Zwischenfelder 20, 21 weggelassen ist. Letzteres vor allem deshalb, weil aufgrund der Aussparungen an den Rändern der Zwischenfelder 19 bis 22 im oberen Teil des Abfallbehälters 31 nur zwei Lagen vorhanden sind, nämlich das Seitenwandfeld 8 und Teile der Zwischenfelder 20, 21. Die Höhe der dazwischen liegenden dritten Lage (vgl. den unteren Teil der Darstellung gemäß Fig. 3 mit den Bezugszahlen 19, 22) ist in Fig. 2 mit einer strichpunktierten Linie 32 veranschaulicht.

Der aus Fig. 2 ersichtliche Rahmen 33, der im Querschnitt eine U-förmige Gestalt hat, ist in seiner U-Form den Lagestärken so angepaßt, daß er auf den Abfallbehälter 31 aufgedrückt die ein- und zweilagigen Seitenwände 7 bis 10 des Abfallbehälters 31 fest übergreift und die Schnittkanten des Zuschnittes 1 dachartig abdeckt. Zur Schaffung einer ausreichenden Flüssigkeitsdichtigkeit ist vorgesehen, daß die oberen Schnittkanten z.B. mit einer klebenden Dichtmasse abgedeckt werden, die auch den Rahmen 33 festlegt.

Da die Seitenwandfelder 8, 10 durch die Mehrlagigkeit wirkungsvoll versteift sind, ist die Richtung der Wellen 34 des Zuschnittes 1 so gewählt, daß die Wellen 34 bei den Seitenwandfeldern 7 und 9 waagerecht verlaufen. Daß auch der Rahmen 33 eine randverstärkende Wirkung hat, versteht sich von selbst. Zu bemerken ist ferner, daß die Aussparungen an den Rändern der Zwischenfelder 19 bis 22 so gewählt sind, daß eine Art Verzahnung der Felder 19 bis 22 entsteht, wie dies die Stirnseite der Fig. 2 erkennen läßt. Dort sieht man auch, welche Wirkung die Sicherungslappen 27, 30 haben.

Für die geforderte absolute Dichtigkeit des Abfallbehälters 31 kann z.B. der Zuschnitt 1 mit einem Spezialpapier 35 (vgl. Fig. 1) versehen sein, das entweder zur Wellpappe selbst gehört oder zusätzlich aufgebracht ist. Ähnliche Wirkung kann auch mit sattem Imprägnieren erreicht werden. Um die vom Bodenfeld 2 ausgehenden und nach oben strebenden Eckbereiche 36, 37, 38 und 39 flüssigkeitsdicht zu machen, wird in diesen Bereichen eine z.B. klebende Dichtungsmasse eingebracht, wie dies im oberen inneren Teil der Fig. 3 durch zwei Wülste angedeutet ist.

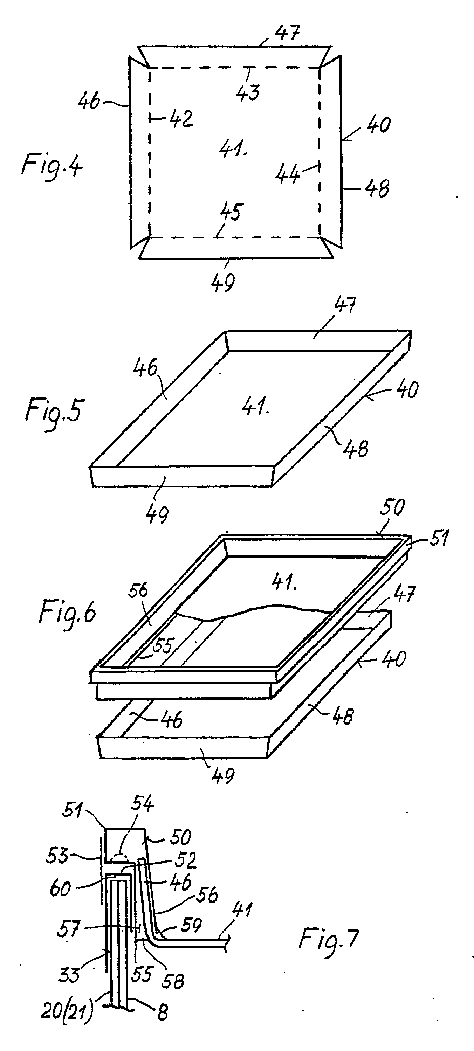

Gemäß Fig. 4 besteht ein Deckelzuschnitt 40 aus einem Feld 41, das über Faltlinien 42, 43, 44 und 45 in Randstreifen 46, 47, 48 und 49 übergeht, die in den Eckbereichen so ausgeklinkt sind, daß sie im aufgestellten Zustand leicht nach schräg außen ragen, wie dies aus den Fig. 5 und 6 ersichtlich ist.

Die Fig. 6 zeigt über dem Deckelzuschnitt 40 einen Deckelrahmen 50, der sich besonders gut aus Fig. 7 ergibt. Dieser Deckelrahmen 50 weist einen umlaufenden leistenartigen Vorsprung 51 auf, der bei aufgesetztem Deckel 40, 50 auf den Abfallbehälter 31 auf der umlaufenden schmalen Fläche 52 des Behälterrahmens 33 zur Auflage kommt. Die Verbindung der beiden Rahmen 33, 50 kann mit einem Klebeband 53 erfolgen, was aber nicht ausschließt, daß die beiden Rahmen miteinander verklebt werden könnten. Es ist aber auch denkbar, in einen der beiden aufeinander zu liegen kommenden Flächen der Rahmen 33, 50 eine Hohlkehle 54 vorzusehen, in die sich eine endlose klebende Dichtschnur einlegen läßt.

Der Deckelrahmen 50 bildet zwischen zwei Schenkeln 55, 56 einen umlaufenden Spalt 57, der mit einer Dichtmasse ausgefüllt wird, in die sich die abgekanteten Randstreifen 46 bis 49 (hier 46) eindrücken lassen. Das verdrängte Material läßt sich dann zu Dichtstreifen 58, 59 verstreichen. Da auch das Seitenwandfeld 8 und auch die Teile 20 bzw. 21 mit Dichtmasse 60 in dem Behälterrahmen 33 festgelegt sind, kann kein Zweifel darüber bestehen, daß der Behälter 31 zusammen mit seinem Deckel 40, 50 einen absolut sicheren Abfallbehälter 31 darstellt, mit dem selbst gefährliche Abfälle bedenkenlos zu einer Entsorgungsanlage gebracht werden können.

## Ansprüche

1. Abfallbehälter, insbesondere für anstekkungsgefährliche Abfälle oder für sonstige gefährliche Stoffe, der eine beliebige Gestalt aufweist und mit einem Deckel verschließbar ist, dadurch gekennzeichnet, daß ein einziger aus einem Werkstoff auf Holzbasis, z.B. aus Wellpappe hergestellter Zuschnitt (1) um Faltlinien (3 - 6, 11 - 18 und 23 - 26) zu dem Behälter (31) aufstellbar ist, wobei der Zuschnitt (1) zumindest an derjenigen Fläche eine flüssigkeitsdichte Schicht (vgl. 35), Beschichtung, Imprägnierung o. dgl. aufweist, welche die Boden- und Innenflächen des Behälters (31) ergibt, und die von einem z.B. quadratischen Bodenfeld (2) nach oben strebenden Eckbereiche(36 - 39) des Behälters (31) mit einer flüssigkeitsdichten Masse, insbesondere mit einer klebenden Dichtungsmasse ausgekleidet sind und der obere Rand des Behälters (1) für die Aufnahme eines abdichtenden Deckels (40, 50) hergerichtet ist.

2. Abfallbehälter nach Anspruch 1, dadurch gekennzeichnet, daß der z.B. mit einem Spezialpapier (vgl. 35) im Kraftliner-Polyäthylen-Kraftliner-Verbund hergestellte flüssigkeitsdichte Zuschnitt (1) ein viereckiges Bodenfeld (2) aufweist, das über Faltlinien (3 - 6) in sich gfs. konisch erweiternde Seitenwandfelder (7 - 10) übergeht, die ebenfalls über Faltlinien (11 - 18) sich nach den Seiten hin in die Seitenwandfelder (7 - 10) miteinander verbindende Zwischenfelder (19 - 22) fortsetzen, die ihrerseits von den Ecken des Bodenfeldes (2) ausgehend etwa diagonal faltbar (vgl. 23 - 26) sind.

3. Abfallbehälter nach den Ansprüchen 1 und 2, dadurch gekennzeichnet, daß die diagonal faltbaren Zwischenfelder (19 - 22) derart ausgespart sind, daß sie bei aufgestelltem Zuschnitt (1) etwa in der oberen Hälfte zweier einander gegenüberliegender Außenseiten (vgl. 8, 10) des Behälters (31) in einer Ebene liegen.

4. Abfallbehälter nach Anspruch 3, dadurch gekennzeichnet, daß die aufeinander zu liegen kommenden Teile der diagonal faltbaren Zwischenfelder (19 - 22) durch angestanzte und nach innen geschlagene Lappen (27 - 30) gegen Auffedern gesichert sind.

5. Abfallbehälter nach den Ansprüchen 1 bis 4, dadurch gekennzeichnet, daß die oberen Schnittkanten des aufgestellten Zuschnittes (1), also des Behälters (31) abgedichtet sind und diese beliebig gestaltete Abdichtung gleichzeitig eine Lagesicherung der diagonal gefalteten (vgl. 23 - 26) und an zwei einander gegenüberliegenden Seitenwandfeldern (vgl. 8, 10) zur Anlage kommenden Zwischenfelder (19 - 22) darstellt.

6. Abfallbehälter nach Anspruch 5, dadurch gekennzeichnet, daß für die Abdichtung und Lagesicherung ein im Querschnitt U-förmiger, nach unten offener und den jeweiligen ein-, zwei- oder dreilagigen Stärken der Seitenwandfelder (8, 10) angepaßter Rahmen (33) aus Kunststoff, Metall, Blech o. dgl. vorgesehen ist, der z.B. mit einer klebenden Dichtungsmasse festgelegt ist und gleichzeitig das Widerlager (52) für den Deckel (40, 50) des Behälters (31) bildet.

7. Abfallbehälter nach den Ansprüchen 1 bis 6, dadurch gekennzeichnet, daß der abdichtende Deckel (40, 50) aus dem gleichen Werkstoff wie der Zuschnitt (1) für den Behälter (31) besteht und von einem Rahmen (50) aus Kunststoff, Metall, Blech o. dgl. flüssigkeitsdicht eingefaßt ist, der einmal passend in den Rahmen (33) oder in die anders gestaltete Abdichtung des Behälters (31) eingreift und zum anderen sich mit einem umlaufenden Vorsprung (51) auf dem Rahmen (33) bzw. der Abdichtung des Behälters (31) abstützt.

8. Abfallbehälter nach Anspruch 7, dadurch gekennzeichnet, daß die Außenflächen des Vorsprunges (51) und die Außenflächen des Behälterrahmens (33) in gleichen Ebenen liegen und zur Aufnahme eines Haftklebebandes (53) dienen.

9. Abfallbehälter nach Anspruch 7, dadurch gekennzeichnet, daß zumindest eine der aufeinander zu liegen kommenden Flächen der beiden Rahmen (z.B. 50) hohlkehlartig (vgl. 54) ausgeführt ist und eine endlose und klebende Dichtschnur dieser Gestaltung angepaßt ist.

10. Abfallbehälter nach Anspruch 7, dadurch gekennzeichnet, daß die aufeinander zu liegen kommenden, gfs. schräg liegenden Flächen der beiden Rahmen (33, 50) miteinander dicht verklebt sind.

11. Abfallbehälter nach den Ansprüchen 1 bis 10, dadurch gekennzeichnet, daß der Rahmen (50) des Deckels (40, 50) im Querschnitt einen nach unten offenen umlaufenden, gfs. konischen Spalt (57) bildet, in den um Faltlinien (42 -45) aufgestellte, gfs. leicht nach außen gerichtete Randabkantungen (46 - 49) des Deckelzuschnittes (40) abgedichtet und lagesicher eingreifen.

12. Abfallbehälter nach Anspruch 11, dadurch gekennzeichnet, daß das Abdichten und Lagesichern der Randabkantungen (46 - 49) des Deckelzuschnittes (40) mit einer klebenden Dichtmasse erfolgt, die sich in dem Spalt (57) befindet und beim Eindrücken der Randabkantungen (46 - 49) derart verdrängt wird, daß im Bereich der unteren Ränder des Rahmens (50) die austretende Dichtmasse verstreichbare Dichtstreifen (58, 59) bildet.

Fig.1

Fig.2

Fig.3

Fig.4

Fig.5

Fig.6

Fig.7

**Europäisches Patentamt**

# EUROPÄISCHER RECHERCHENBERICHT

Nummer der Anmeldung

EP 89 10 2982

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | DE-U-8 608 885 (KAYSER) <br> * Seite 5, Absätze 1,2; Seite 10, Zeile 7 - Seite 12, Zeile 26; Figuren * <br> --- | 1,2,3 | B 65 F 1/00 <br> B 65 D 5/24 <br> B 65 D 5/44 <br> B 65 D 5/64 |
| Y | FR-A-2 178 687 (ILFORD LTD) <br> * Seite 4, Zeilen 20-24; Seite 4, Zeile 31 - Seite 5, Zeile 5; Figuren 1,3 * <br> --- | 1,2,3 | |
| Y | FR-A-2 158 908 (SODER) <br> * Seite 2, Zeile 27 - Seite 3, Zeile 5; Seite 4, Zeile 18 - Seite 5, Zeile 1; Figuren 1,2a,b,c * | 3 | |
| A | <br> --- | 4 | |
| A | GB-A-1 108 338 (MARDON) <br> * Insgesamt * <br> --- | 4 | |
| A | FR-A-2 145 283 (UNILEVER) <br> * Seite 2, Zeilen 31-33; Seite 3, Zeilen 3-17; Figuren 2,3 * <br> --- | 5,6 | |
| A | DE-A-3 319 037 (RAUM) <br> * Seite 8, Zeilen 1,2; Seite 9, Zeile 9 - Seite 10, Zeile 5; Figuren 4-7 * <br> --- | 5,6 | **RECHERCHIERTE SACHGEBIETE (Int. Cl.4)** <br><br> B 65 F <br> B 65 D |
| A | US-A-2 464 278 (WILSON) <br> * Spalte 3, Zeile 64 - Spalte 4, Zeile 3; Spalte 4, Zeilen 36-58; Spalte 5, Zeilen 40-50; Figuren 1-4,6 * <br> --- | 7,8,10, 11 | |
| A | FR-A-2 320 238 (SOFHUNIC) <br> * Seite 6, Zeilen 8-34; Figuren 6-8 * <br> --- | 8 | |
| A | GB-A-1 475 063 (AIRFIX) <br> --- | | |
| A | FR-A-2 382 374 (BERTOLUCCI) <br> ----- | | |

Der vorliegende Recherchenbericht wurde für alle Patentansprüche erstellt

| Recherchenort | Abschlußdatum der Recherche | Prüfer |
|---|---|---|
| DEN HAAG | 02-06-1989 | MARTENS L.G.R. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
...................................................................
& : Mitglied der gleichen Patentfamilie, übereinstimmendes Dokument

EPO FORM 1503 03.82 (P0403)